# EUROPEAN PATENT SPECIFICATION

(11) **EP 3 500 102 B1**
(45) Date of publication and mention of the grant of the patent: **07.06.2023**
(21) Application number: 17758255.8
(22) Date of filing: 15.08.2017
(51) Int. Cl.: A01P 1/00, A61L 2/16, A61L 2/18, A01N 33/12, A01N 47/44, A01N 37/44, A61K 8/02, A61K 31/14

(54) **ANTI-MICROBIAL COMPOSITION**
ANTIMIKROBIELLE ZUSAMMENSETZUNG
COMPOSITION ANTIMICROBIENNE

(30) Priority: 17.08.2016 GB 201614087
(43) Date of publication of application: 26.06.2019
(73) Proprietor: Byotrol PLC, Manchester M40 7RU (GB)
(72) Inventor: EVANS, Huw, Chester CH2 4NU (GB); PLUMMER, Christopher, Chester CH2 4NU (GB); LEE, Monica, Chester CH2 4NU (GB); MCINERNEY, Rose, Chester CH2 4NU (GB); SOLOMOM-DENSON, Karla, Chester CH2 4NU (GB); HURD, Rhiannon Sian, Tarporley Cheshire CW6 9PL (GB)
(74) Representative: Potter Clarkson
(86) International application number: PCT/GB2017/052399
(87) International publication number: WO 2018/033718

(56) References cited:
- WO-A1-2013/061082
- WO-A1-2013/098547
- WO-A1-2015/145100
- WO-A2-2013/076697
- GB-A- 2 518 607
- GB-A- 2 533 527
- US-A1- 2002 173 775
- DATABASE WPI Week 200143 25 April 2001 (2001-04-25) Thomson Scientific, London, GB; AN 2001-408799 XP002774605, "Topical antimicrobial composition useful for combating bacterial and fungal infections comprises a quaternary ammonium salt, a chlorhexidine salt and an antiseptic agent", & ZA 200 004 781 A (MARAIS O M J F) 25 April 2001 (2001-04-25)

## Description

This invention relates to anti-microbial compositions and to formulations including the anti-microbial compositions. In particular, the invention relates to anti-microbial compositions that can be used on skin.

Micro-organisms such as bacteria and viruses, for example norovirus, polio virus and adenovirus, are well known to present health hazards due to infection or contamination. Along with fungi, yeasts and other micro-organisms they can also cause spoilage of items such as food, clothing and produce unpleasant odours. When micro-organisms are present on the surface of a substrate, such as skin, they can replicate rapidly to form colonies.

Many anti-microbial agents can destroy micro-organisms that are present in a wide range of environments. For example, anti-microbial agents that may be used in medical, industrial, commercial, domestic and marine environments are known. Many of the known anti-microbial agents have previously been included in compositions for use in these environments for various applications.

Known anti-microbial agents and the compositions that contain these anti-microbial agents act via a number of different mechanisms.

For example, many anti-microbial agents are poisonous to microorganisms and, therefore, destroy microorganisms with which they are contacted. Examples of this type of anti-microbial agent include hypochlorites (bleaches), phenol and compounds thereof, and salts of copper, tin and arsenic.

However, whilst many of these anti-microbial agents are effective against micro-organisms and appropriate for certain environments, their mechanisms can make them unsuitable or disadvantageous for topical application to human skin due to their detrimental effects to human health or on skin condition. For example, hypochlorite bleach is very effective at killing micro-organisms on solid surfaces, but inappropriate for application to skin due to its corrosive properties. Also, these materials tend to be effective in a wet environment for sterilization and cleansing but stop working shortly after drying.

Many skin sanitizers are alcohol-based. These are typically an alcohol-containing preparation designed for application to the hands for reducing the number of viable microorganisms on the hands. Such preparations typically contain 60%-95% ethanol or isopropanol. However, while isopropyl alcohol has established anti-bacterial properties, it has the disadvantage that, when used regularly, it can cause dryness and skin irritation. As a result, some people may be reluctant to use such creams, soaps and other compositions comprising significant levels of isopropanol.

Some anti-microbial agents are highly toxic to humans and animals and are dangerous to handle. Specialist handling, treatment and equipment are therefore required in order to handle them safely. The manufacture and disposal of compositions comprising this type of anti-microbial agent can, therefore, be problematic. There can also be problems associated with the use of compositions containing this type of anti-microbial agent, particularly in consumer materials where it is difficult to ensure that they are used for designated purposes.

Herein, unless the context indicates otherwise, "toxicity" is intended to refer to toxicity to complex organisms such as mammals. References to "toxic" are to be construed accordingly.

Increasing scrutiny of the environmental credentials of chemicals is further restricting the use of anti-microbial agents not just for topical application on skin but for general use. For example, some anti-microbial agents once they enter the environment can harm other organisms. They can also be very stable and persist in the environment for long periods causing concerns around build-up and residual levels.

Because of these factors the list of effective and available anti-microbial agents, particularly for use on skin, is becoming more restricted and the burden of registering new anti-microbials more prohibitive. At the same time, the public are becoming increasingly concerned with sanitization, both of person and property following outbreaks such as MRSA in hospitals.

There is a need to provide compositions for a variety of applications and uses, such as compositions for use on skin, for example hand sanitising agents, that have anti-microbial properties and that address one or more of the problems set out above. However, it is not a straight forward matter to do this. There are regulations such as the Biocidal Products Regulations (Directive 98/8/EC) which regulates the use of anti-microbial agents both in terms of the nature and the amount of a given anti-microbial agent that may be used. Additionally, the potential reactivity of an anti-microbial agent once in a composition is important as some anti-microbial agents are rendered inactive by chemical reaction. Even where an anti-microbial agent is not deactivated by chemical reaction it may have its activity suppressed by other components of the composition.

The present inventors have surprisingly found that the foregoing deficiencies can be overcome by certain combinations of components. It has also been found that compositions containing these combinations of components can have some surprising and unexpected properties.

GB 2 533 527 describes an antimicrobial composition with residual effect based on:
(i) an amino acid, a derivative of an amino acid, a salt of an amino acid, or a salt of a derivative of an amino acid or a mixture thereof;
(ii) an anti-microbial component comprising two or more anti-microbial quaternary ammonium compounds or at least one anti-microbial quaternary ammonium compound and chlorhexidine or a chlorhexidine salt; and
(iii) a polar solvent.

WO 2013/076697 describes a three-component anti-microbial blend comprising undecylenoyl compounds, e.g. undecylenamidopropyltrimonium metasulphate.

The present invention provides an anti-microbial composition comprising:
(i) a compound of formula:

   [N(CH₃)₃Q²]⁺Y⁻ (C²)

   wherein Q² has structure -(CH₂)ₐNHC(O)(CH₂)_{b}CHCH₂, wherein a + b is an integer of from 8 to 18, provided that a and b are each independently an integer of from 1 to 9, wherein Y⁻ is a halide anion, alkylsulphate, sulphonate, saccharinate, or bicarbonate, or a mixture of compounds of formula (C²);
(ii) at least one anti-microbial quaternary ammonium compound and chlorhexidine or a chlorhexidine salt; and
(iii) a polar solvent.

### Component (i)

Component (i) comprises one or more compounds of formula:

[N(CH₃)₃Q2]⁺Y⁻ (C²)

wherein Q² has structure -(CH₂)ₐNHC(O)(CH₂)_{b}CHCH₂, wherein a + b is an integer of from 8 to 18 (such as 10 to 18), provided that a and b are each independently an integer of from 1 to 9, for example a is from 1 to 5 and b is from 5 to 9, wherein Y⁻ is as defined above, for example Y⁻ is an alkylsulphate anion, such as methosulphate anion.

An example of the group Q² is -(CH₂)₃NHC(O)(CH₂)₈CHCH₂

An example of a compound of formula C² that may be used in the present invention is [N(CH₃)₃(CH₂)₃NHC(O)(CH₂)₈CHCH₂]⁺ CH₃SO₄⁻, undecylenamidopropyltrimonium methosulfate (available commercially as Rewocid UTM 185).

### Component (ii)

The compositions of the invention comprise chlorhexidine or a chlorhexidine salt. Examples of suitable chlorhexidine salts include the acetates, formates, gluconates, hydrochlorides, isoethionates, lactates, and succinamates of chlorhexidine. For example, suitable salts include, but are not limited to chlorhexidine diphosphanilate, chlorhexidine digluconate, chlorhexidine diacetate, chlorhexidine dihydrochloride, chlorhexidine dichloride, chlorhexidine dihydroiodide, chlorhexidine diperchlorate, chlorhexidine dinitrate, chlorhexidine sulfate, chlorhexidine sulfite, chlorhexidine thiosulfate, chlorhexidine di-acid phosphate, chlorhexidine difluorophosphate, chlorhexidine diformate, chlorhexidine dipropionate, chlorhexidine di-iodobutyrate, chlorhexidine di-n-valerate, chlorhexidine dicaproate, chlorhexidine malonate, chlorhexidine succinate, chlorhexidine succinamate, chlorhexidine malate, chlorhexidine tartrate, chlorhexidine dimonoglycolate, chlorhexidine mono-diglycolate, chlorhexidine dilactate, chlorhexidine dialpha-hydroxyisobutyrate, chlorhexidine diglucoheptonate, chlorhexidine di-isothionate, chlorhexidine dibenzoate, chlorhexidine dicinnamate, chlorhexidine dimandelate, chlorhexidine di-isophthalate, chlorhexidine isoethionate chlorhexidine di-2-hydroxy-napthoate, and chlorhexidine embonate.

A specific example of a chlorhexidine salt that may be used in the compositions of the invention is chlorhexidine di-gluconate.

Component (ii) of the compositions of the invention also comprises at least one quaternary ammonium anti-microbial agent.

The quaternary ammonium anti-microbial agent(s) used in the present invention are typically water soluble at room temperature and pressure.

Suitable anti-microbial quaternary ammonium compounds include compounds of formula (A) shown below. 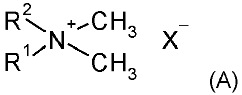 wherein R¹ and R² are each independently a straight chain, unsubstituted and uninterrupted C₈₋₁₂ alkyl group. In one aspect, the groups R¹ and R² contain the same number of carbon atoms but this is not essential and compounds in which R¹ and R² contain different number of carbon atoms can be used. X⁻ is a halide anion (such as chloride, bromide, fluoride, iodide), sulphonate (GSO₃⁻, wherein G is an organic residue, such as a C₁₋₆ alkyl group), saccharinate, or bicarbonate.

In the compounds of formula (A) each group R¹ and R² is independently a straight chain, unsubstituted, uninterrupted C₈₋₁₂ alkyl group, for example an alkyl group containing 8, 9, 10, 11 or 12 carbon atoms. R¹ and R² may be the same, for example R¹ and R² may both be alkyl groups containing 8, 9, 10, 11 or 12 carbon atoms, as an example both R¹ and R² may be 10.

Suitable anti-microbial quaternary ammonium compounds also include benzalkonium compounds having the formula (B) shown below. 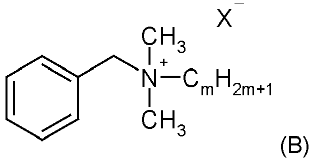 wherein m is from 8 to 18, and X⁻ is a halide anion such as chloride, bromide, fluoride, iodide, sulphonate (GSO₃⁻, wherein G is an organic residue, such as a C₁₋₆ alkyl group), saccharinate or bicarbonate.

The compounds of formula (B) are generally called benzalkonium compounds. The benzalkonium chloride is provided and/or used as a mixture of C₈₋₁₈ alkyl groups, particularly a mixture of straight chain, unsubstituted and uninterrupted alkyl groups n-C₈H₁₇ to n-C₁₈H₃₇, mainly n-C₁₂H₂₅ (dodecyl), n-C₁₄H₂₉ (tetradecyl), and n-C₁₆H₃₃ (hexadecyl). Preferably m is 8, 10, 12, 14 and/or 16. Most preferably m is from 8 to 12 for example 8, 10 and/or 12 or from 12 to 16, for example 12, 14 and/or 16.

By benzalkonium compounds we also include derivatives of benzalkonium compounds whereby the alkyl group CₘH₂ₘ₊₁ shown below can be substituted by another organic group. Such organic groups include but are not limited to alkenyl, phenyl, amide, ester, alcohol, and ether groups or combinations thereof.

An example of such a derivative of a benzalkonium compound is diisobutylphenoxyethoxyethyl-dimethylbenzyl ammonium chloride, whose INCI name is benzethonium chloride (typically abbreviated to BENZ). The structure of benzethonium chloride is shown below.

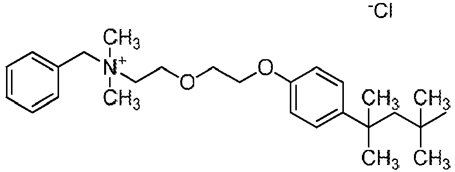

In an aspect of the invention, where the composition comprises two or more anti-microbial quaternary ammonium compounds, a compound of formula (A) and a compound of formula (B) may be present, or the composition may comprise more than one compound of formula (A) or more than one compound of formula (B).

Examples of quaternary ammonium compounds of formula (A) include di-n-decyldimethyl ammonium chloride, octyl decyl dimethyl ammonium chloride and dioctyl dimethyl ammonium chloride.

Examples of commercially available compounds of formula (A) include Acticide DDQ 40 from Thor, Bardac 2240 from Lonza, Bardac 2280 from Lonza and Maquat 4480E, from Mason Chemical Company, USA.

Examples of quaternary ammonium compounds of formula (B) include N,N-benzyldimethyloctylammonium chloride, N,N-benzyldimethyldecylammonium chloride , N-dodecyl-N-benzyl-N,N-dimethylammonium chloride, N-tetradecyl-N-benzyl-N,N-dimethylammonium chloride, N-hexadecyl-N,N-dimethyl-N-benzylammonium chloride, N,N-dimethyl N-benzyl N-octadecyl ammonium chloride and mixtures thereof.

It will be appreciated that a single CAS number often refers to more than one blend or mixture. A CAS classification for a commercial preparation typically covers blends comprising specified compounds in amounts within defined ranges. The compositions having the CAS numbers quoted above are only examples of compositions having a given CAS number that may be used in the present invention.

Examples of commercially available compounds of formula (B) include Acticide BAC 50M from Thor, Barquat MB50/80 from Lonza, Lonzagard Benzethonium Chloride USP from Lonza.

Typically, the anti-microbial quaternary ammonium compound or compounds used in the composition of the invention is selected from the group consisting of di-n-decyldimethyl ammonium chloride (DDAC (CAS number 7173-51-5)), benzalkonium chloride (BAC) such as benzalkonium chloride of formula (B) above wherein m is 8 and mixtures thereof. This benzalkonium chloride has CAS number 68424-85-1, and benzethonium chloride (BENZ).

The amount of component (i) and anti-microbial component (ii) present in the compositions of the present invention will vary depending on a number of factors, such as the intended use of composition and the particular compound(s) used.

For example, as will be appreciated by the person skilled in the art, higher concentrations of (i) and/or (ii) may be required in order to provide sterilisation compared to providing sanitisation or disinfection.

Anti-microbial component (ii) may consist essentially of at least one anti-microbial quaternary ammonium compound and chlorhexidine or a chlorhexidine salt.

Anti-microbial component (ii) may consist of at least one anti-microbial quaternary ammonium compound and chlorhexidine or a chlorhexidine salt.

The ratio of the at least one anti-microbial quaternary ammonium compound to chlorhexidine or a chlorhexidine salt is preferably from 1:1 to 3:1 in the compositions of the invention.

A composition of the invention may, for example, comprise as component (i) a compound of formula:

[N(CH₃)₃Q²]⁺Y⁻ (C²)

wherein Q² has structure -(CH₂)ₐNHC(O)(CH₂)_{b}CHCH₂, wherein a + b is an integer of from 8 to 18 (such as 10 to 18), provided that a and b are each independently an integer of from 1 to 9, for example a is from 1 to 5 and b is from 5 to 9, wherein Y⁻ is as previously defined and;
as component (ii) an anti-microbial quaternary ammonium compound of formula (A) 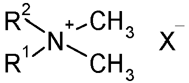 wherein R₁ and R₂ are each independently a straight chain, un-substituted and uninterrupted C₈₋₁₂ alkyl group and X⁻ is a halide anion such as chloride, bromide, fluoride, iodide or sulphonate, saccharinate, or bicarbonate and chlorhexidine or a chlorhexidine salt. Optionally, these compositions may contain a compound of formula (B) as defined above.

The composition of the invention may, for example, comprise as component (i) [N(CH₃)₃(CH₂)₃NHC(O)(CH₂)₈CHCH₂]⁺ CH₃SO₄⁻, (undecylenamidopropyltrimonium methosulfate) and;
as component (ii) an anti-microbial quaternary ammonium compound of formula (A) 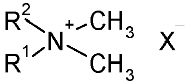 wherein R₁ and R₂ are each independently a straight chain, un-substituted and uninterrupted C₈₋₁₂ alkyl group and X⁻ is a halide anion such as chloride, bromide, fluoride, iodide or sulphonate, saccharinate, or bicarbonate and chlorhexidine or a chlorhexidine salt. Optionally, these compositions may contain a compound of formula (B) as defined above.

Examples of the compound of formula (A) suitable for use in such compositions include din-decyldimethyl ammonium chloride, octyl decyl dimethyl ammonium chloride, dioctyl dimethyl ammonium chloride and mixtures thereof.

Examples of the compound of formula (B) suitable for use in such compositions include benzalkonium chloride (BAC) and/or benzethonium chloride (BENZ).

The compositions of the invention may additionally comprise other suitable anti-microbial agent(s) (b), such as those described in the EPA (United States Environmental Protection Agency) Listing and Annex I and Annex 3 of the EC Biocides Directive.

Typically, the compounds used as anti-microbial agents are those that are suitable for application to the skin. For example, compounds that meet the required regulatory approval for human hygiene, such as those compounds approved by the European Commission, when used in products of Product-type 1 as listed in Annex II of the European Commission's delegated regulation (EU) No. 1062/2014 of 4 August 2014 and/or those approved by the FDA (Food and Drugs Administration).

Suitable additional anti-microbial agents (b) that may be included in the anti-microbial component (ii) include anti-microbial agents that are not quaternary ammonium compounds. Preferably, these additional anti-microbial agent(s) are water soluble at room temperature and pressure.

Examples of suitable additional anti-microbial agents include but are not limited to polymeric biguanidines (e.g. polyhexamethylene biguanidine (PHMB)), hydrogen peroxide, lactic acid, silver, salts of silver (for example silver chloride, silver carbonate, silver citrate, silver dihydrogen citrate), octenidine HCl, amphoteric compounds, iodophores, phenolic compounds, amine anti-microbial agents and nitrogen based heterocyclic compounds, ortho phenyl phenol (OPP), and nitro bromopropanes (e.g. bronopol (INN), 2-bromo-2-nitropropane-1,3-diol), naturally derived biocidal compounds (e.g. honey and extracts of honey such as those comprising methyl glyoxal), flavenoid based anti-microbials, and essential oils.

In one aspect, the compositions of the invention are free of inorganic anti-microbial agents such as those comprising silver. In this aspect, any additional anti-microbial agent is an organic anti-microbial agent.

In one aspect, the compositions of the invention do not comprise PHMB or they may be free of polymeric biguanides. For example, they do not contain a biguanide in addition to chlorhexidine.

In one aspect of the invention, the anti-microbial composition does not comprise any isothiazalones and/or any nitrobromopropanes such as bronopol and/or any hypochlorites.

In one aspect of the invention, the anti-microbial composition does not contain anti-microbial compounds that are poorly soluble in water such as phenolic compounds.

Typically, when formulated as a ready to use composition, the amount of component (i) may be from about 0.01 to 5% by weight of the composition, for example from about 0.05 to about 2% by weight of the composition, such as from about 0.08 to about 0.5% by weight of the composition.

Typically, when formulated as a ready to use composition, the total amount of anti-microbial component (ii) and component (b) if present (ie the combined amount of anti-microbial quaternary ammonium compound and chlorhexidine or a salt thereof and any additional anti-microbial component (b)), may be from about 0.01 to about 5% by weight of the composition, for example from about 0.02 to about 2% by weight of the composition, such as from about 0.05 to about 0.6% by weight of the composition.

When the composition of the invention is formulated for use as a hand sanitizer, the amount of anti-microbial component (ii) may be from about 0.05 to about 0.6% by weight.

The anti-microbial component (ii) may comprise equal amounts of quaternary ammonium compound and chlorhexidine, or salt thereof. For example, if the total amount of anti-microbial component is about 0.2% by weight of the composition, the amount of quaternary ammonium compound would be about 0.1% and the amount of chlorhexidine or salt thereof would be about 0.1%. Alternatively, the anti-microbial component may comprise different amounts of quaternary ammonium compound and chlorhexidine. For example, the ratio of the quaternary ammonium compound to the chlorhexidine or salt thereof may be from about 4:1 to 1:2, preferably from 1:1 to 3:1.

Typically, in the compositions of the invention, the ratio of component (i) to anti-microbial component (ii) ranges from 15:1 to 1:10 or from 5:1 to 1:5.

Examples of compositions of the invention include those wherein the ratio of the at least one anti-microbial quaternary ammonium compound to chlorhexidine or a chlorhexidine salt is from 1:1 to 3:1 and the ratio of component (i) to component (ii) is from 15:1 to 1:10 or from 5:1 to 1:5. In such compositions, the amount of component (i) may be from about 0.01 to about 5% by weight of the composition and the amount of component (ii) and component (b) if present may be from about 0.05 to about 0.6% by weight of the composition.

The compositions of the invention comprise a polar solvent (iii). Suitable polar solvents include, but are not limited to, water, alcohols, glycol ethers and mixtures thereof.

Suitable alcohols include, but are not limited to, straight or branched chain C₁ to C₅ alcohols, (such as methanol, ethanol, n-propanol, iso-propanol, mixtures of propanol isomers, n-butanol, sec-butanol, tert-butanol, iso-butanol), mixtures of butanol isomers, 2-methyl-1-butanol, n-pentanol, mixtures of pentanol isomers and amyl alcohol (mixture of isomers), and mixtures thereof.

Preferred polar solvents for use in the compositions of the invention include, but are not limited to, water, ethanol, isopentydiol, mono propylene glycol, hexylene glycol, 3-methoxy-3-methyl-1-butanol (MMB), n-propanol, isopropanol, ethylene glycol ethers, propylene glycol ethers, butyl diglycol (BDG) and mixtures thereof. In one aspect, the composition comprises water, or a mixture of water and one or more alcohols selected from the alcohols described above. In such mixtures, water is preferably the major component. The polar solvent may consist essentially of water or consist of water.

If the compositions of the invention comprise an alcohol, the alcohol is typically present in an amount lower than the amount necessary for the alcohol to provide an anti-microbial effect, but at a level that it is believed improves drying of the composition on skin. For example, the compositions of the invention may comprise up to about 30% by weight alcohol, such as between 20 and 30% by weight alcohol or less than about 20% by weight alcohol, such as from about 15 to about 20% by weight alcohol.

It will be appreciated that the percentage of alcohol may depend on the use of the composition. For example, when used on a wipe, the composition of the invention may comprise up to 20% alcohol.

In one aspect, the compositions of the invention are substantially free of alcohol. For example, the compositions may contain 1% or less by weight alcohol. For example, the compositions may contain less than 1% or less than 0.5% by weight or 0.1% by weight or less of an alcohol such as isopropanol. As an example, compositions of the invention may comprise no isopropanol or may comprise no alcohol.

In another aspect, the compositions of the invention may comprise an alcohol up to a level of 70% by weight, e.g. up to about 50% by weight.

The composition may comprise water or a mixture of water and one or more alcohols selected from the alcohols described above. In such mixtures, water is preferably the major component.

For example, the polar solvent may consist of or consist essentially of water. As another example, the polar solvent may be water and from about 0 to about 20% by weight, such as from about 15 to about 20% by weight of an alcohol, for example ethanol or isopropanol.

The compositions of the invention can be provided in concentrated form for dilution before use or in ready to use form. It is preferred that the compositions of the invention are provided in ready to use form and, unless otherwise stated, information about amounts (such as weight% or ppm) provided in this document relate to ready to use compositions.

The pH of the compositions and formulations used can vary within wide limits, for example from about pH 2 to about pH 12, more preferably from about pH 3 to about pH 10 or about pH 4.5 to 8. The pH of a formulation used in the invention may be similar to that of known formulations which are intended to be used for the same purpose or a similar purpose. For example, a formulation that is intended to come into contact with the skin such as a personal care or first aid formulations will typically have a pH which will not irritate the skin, for example from about pH 3 to about pH 8, such as from about pH 3.5 to about pH 7.5 or from about pH 4 to about pH 7.

When used as a hand sanitizer, the pH of the compositions of the invention is typically from about 4 to about 7.

It will be appreciated that the compositions of the invention can comprise other ingredients commonly used in the art. The nature of any other ingredients used will depend on the nature and intended purpose of the composition. The person of ordinary skill in the art will know which additional ingredients are suitable for use in compositions for different applications.

The compositions of the present invention may comprise additional materials such as surfactants, complexing agents, buffering agents, thickening agents, skin conditioning compounds, polar solvents and fragrances.

The selection of surfactants will depend on the nature of and the intended purpose of the composition. Suitable surfactants for use in formulations intended for different purposes will be within the knowledge of the person of ordinary skill in the art. Likewise, the selection of suitable amounts of surfactant will be within the knowledge of the person of ordinary skill in the art. Suitable surfactants may be selected from non-ionic, cationic or amphoteric and mixtures thereof.

Some compositions of the invention may comprise a non-ionic surfactant. Suitable non-ionic surfactants include, but are not limited to, amine oxides, alkyl polyglucosides, linear and branched 1° and 2° alcohol ethoxylates, and ethoxylated/propoxylated (EOPO) block polymers.

In one aspect, the composition is substantially free of or free of anionic surfactant. In another aspect, the compositions of the invention do not comprise an amphoteric surfactant.

Some compositions of the invention may comprise an amphoteric surfactant. Suitable amphoteric surfactants include but are not limited to C₆-C₂₀ alkylamphoacetates or amphodiacetates (such as cocoamphoacetates), C₁₀-C₁₈ alkyldimethyl betaines, C₁₀-C₁₈ alkyl amidopropyldimethyl betaines. Examples include but are not limited to coconut amphoteric surfactant cocoamidopropyl betaine (CAPB) (Surfac B4, CAS 61789-40-9), coco imidazoline betaine, oleo amido propyl betaine, and tall oil imidazoline.

Some compositions of the invention may comprise a cationic surfactant. Suitable cationic surfactants include but are not limited to undecylenamidopropyltrimonium methosulphate and cetrimonium chloride.

Typically, the amount of surfactant present in the compositions of the invention is from about 0.01 to 20% by weight. The amount of surfactant depends on a number of factors such as the pH of the composition and the intended use of the composition.

The compositions of the invention may comprise complexing agents. The terms sequestering agents or chelates are sometimes used interchangeably with the term complexing agents. For the purpose of describing this invention, the term complexing agents include both sequestering agents and chelates. Complexing agents can be used to help provide a clear composition even when the compositions of the invention are used with hard water. Examples of suitable complexing agents include but are not limited to EDTA (Ethylenediaminetetraacetic acid), Gluconate, GLDA (Glutamic acid diacetic acid) - Trade name Dissolvine GL, EDDS (Ethylenediamine-N,N'-disuccinic acid), citrates and gluconates or salts of glutaric, adipic and succinic acids and mixtures thereof. If the complexing agent contains a counter ion that counter ion is preferably metallic. Suitable metallic counter ions include but are not limited to Na, Ca, Fe, K, Zn, Mg and Mn.

Preferred complexing agents are GLDA (Dissolvine) and EDTA.

The complexing agents are typically present in an amount of from about 100 to 10,000 ppm, preferably from about 400 to 3,000 ppm, for example from about 500 to 2,000ppm.

Buffering agents may be included to adjust the pH of the composition to the required value and maintain it at or close to that pH value during storage and use.

Suitable pH modifiers include but are not limited to acids such as citric, sulfamic, hydrochloric, phosphoric, nitric, lactic, formic, acetic glycolic or gluconic acids or other mineral or organic acids or bases such as sodium or potassium hydroxide, triethanolamines and carbonates and mixtures thereof.

Thickening agents can be included to adjust the viscosity of the composition to the required value for such purposes as to make the composition more stable; facilitate application of the composition; or for aesthetic benefits. Examples of thickening agents include but are not limited to hydroxypropylmethylcellulose stearoxy ether supplied commercially by Daido chemical corporation under the tradename Sangelose 90, and a polyquaternium 37 supplied by Rheo Lab under the tradename Kleasol 200ST.

The compositions of the invention may contain skin conditioning compounds. Suitable skin conditioning compounds include, but are not limited to, panthenol, tocopheryl acetate, glycerine, polyquaternium compounds, PEG-7-glyceryl cocoate, and silicones and mixtures thereof. Typically, the compositions of the invention do not comprise mucopolysaccharides.

The compositions of the invention may alternatively or additionally contain salts such as the halides of alkali metals or alkaline earth metals such NaCl or KCI. In some situations, the use of salts can facilitate the formation of a stable composition.

The compositions of the invention may also contain other ingredients that are standard in the art such as colorants, fragrances, emollients, antioxidants and mixtures thereof.

The compositions of the invention may be free of anionic materials. Within the meaning of anionic materials, we do not include counterions that may for present in any of the ingredients used in the invention, such as counterions associated with a quaternary ammonium compound. The compositions of the invention may, for example, be free of anionic polyelectrolytes, such as high molecular weight, anionic mucomimetic polymers, polystyrene sulfonic acid polymers and cationic exchange resins.

It has been found that in use compositions of the invention have advantageous anti-microbial effects, and particularly when applied to the surface of skin. For example, such compositions have an anti-microbial effect when initially applied to a surface (so called "wet kill") and they can also have a residual anti-microbial effect in that they control, reduce or prevent the formation of new microbial colonies at the surface (so called "dry kill") that may otherwise result in the formation of more persistent biofilms.

The compositions of the invention are also resistant to washing with water and to wiping. This means that the compositions of the invention provide a residual anti-microbial effect even when the surface which has been treated is subsequently wiped and/or washed or rinsed with water.

The compositions of the invention are particularly suitable for use as skin or hand sanitizers or on other surfaces. Some compositions are suitable for use on other surfaces. The compositions of the invention typically provide a residual anti-microbial effect.

Such compositions may be used in a wide variety of formats and applications for the purpose of skin sanitization. For example, the compositions of the invention are particularly suitable for formulation into mousses, gels, creams, lotions, liquids, sprays and wipes to be used in body or skin sanitisation, such as hand sanitizers.

When used in a wipe format, the substrates used to prepare the wipes may be made of any suitable woven or non-woven material. Suitable wipe materials include, but are not limited to, non-woven fibrous sheet materials and those made of fibres from natural sources such as wood pulp, and viscose or other cellulose based materials, silk fibres and keratin fibres. The materials comprise from 1 to 100% by weight of a cellulosic material. For example, 100% cellulosic materials such as viscose or wood pulp may be used. Other preferred materials include blends of cellulosic and non-cellulosic materials, such as blend of viscose with synthetic substrates such as polyester or polypropylene. Blended materials may comprise from 1 to 99.9 % by weight of a cellulosic material such as viscose, for example from 20 to 80% by weight or from 30 to 70 % by weight of a cellulosic material such as viscose.

The compositions of the invention can be used on humans and animals for both therapeutic and non-therapeutic purposes. Examples of products for non-therapeutic uses include personal care and personal hygiene products. Examples of products for therapeutic purposes include first aid and skin care products.

Compositions of the invention may also be in the form of a liquid and packed into suitable dispensers to provide a foam, mousse or spray in use. Compositions of the invention may be used for foot hygiene products, including those for direct use on the foot and those for the treatment/deodorisation of footwear, particularly sports footwear. Compositions of the invention can also be used in other personal care products which are used for direct application on skin or hair for example soap, bath and shower products, hair care products including shampoo, and anti-dandruff shampoos, hair conditioners hair styling products such as hair mousses, gels and sprays, skincare products such as shaving products, cosmetics and products for hair removal, deodorant and antiperspirant products, baby products including baby cleaning and cleansing products such as baby bath, soaps, wipes, moisturisers, nappy rash cream, products for cleaning surfaces that have regular and high incidence of infant and baby contact.

Compositions of the invention may be used as hygienic cleansers in pet products for direct application to the skin surface of the animal or its coat or fur for the purpose of eradicating or inhibiting micro-organisms which may otherwise have a detrimental effect on the animal's health, well-being or cleanliness. Such compositions can also be applied to materials such as surfaces which come into contact with animals and to which microorganisms may be transferred from the animal. The benefit of such applications is again to eradicate or inhibit such micro-organisms which otherwise may be transferred to another animal or human and also to control or eliminate the by-products that such organisms may produce such as malodour. Such pet applications may include but not be limited to stain and odour removal sprays, disinfectant sprays and wipes, outdoor hutch cleaners, cage cleaners, anti-bacterial cat litter tray sprays and wipes, dog shampoos and spritzers and dog ear cleaning wipes.

Compositions of the invention may be used for First Aid topical applications to skin to sanitize abrasions or wounds on the surface of the skin to prevent or deter infection, or to prevent minor infections such as athletes' foot, verruca, warts, spot/acne prevention/treatment products. Thus, the invention provides products for these uses, such as wound care or first aid products that comprise a composition of the invention.

Compositions of the invention may also be used for medical applications such as sterilization of catheters, dialysis and other medical equipment; inhibiting or eradicating viruses such norovirus, polio virus or adenovirus; and prevention or eradication of persistent biofilms.

Compositions of the invention may also be used on surfaces other than skin. Examples of applications of the invention to surfaces other than skin include but are not limited to: surface cleaners such as those intended for use in bathrooms, kitchens, living areas; hard floor cleaners; carpet cleaners; furniture cleaners; glass/mirror cleaners; toilet care products including solid toilet cleaners such as rim devices and those designed to be placed in the cistern; liquid toilet cleaners excluding those comprising hypochlorite bleaches; dishwashing products such as washing up liquids and preparations from dishwashing machines such as dishwashing solids (in powder or tablet format) & liquids; Laundry products such as solid detergents (in powder or tablet format); liquid detergents; fabric conditioners and "2 in 1" products comprising detergent and fabric conditioner; cleaning products intended for use outdoors such as those for cleaning for wood, stone, concrete or plastics, for example patio cleaner, garden furniture cleaners/treatments, BBQ cleaners, wall and fence cleaners/treatments; plant sprays such as those intended to remove insects such as aphides from plants; food sprays, such as those suitable for use in food preservation.

For the avoidance of doubt, in this specification when we use the term "comprising" or "comprises" we mean that the composition or formulation or component being described must contain the listed ingredient(s) but may optionally contain additional ingredients. When we use the term "consisting essentially of" or "consists essentially of" we mean that the composition or formulation or component being described must contain the listed ingredient(s) and may also contain small (for example up to 5% by weight, or up to 1% or 0.1% by weight) of other ingredients provided that any additional ingredients do not affect the essential properties of the composition, formulation or component. When we use the term "consisting of" we mean that the composition or formulation or component being described must contain the listed ingredient(s) only. These terms can be applied in an analogous manner to processes, methods and uses.

By "substantially free" we mean that the composition or formulation or component being described contains less than 3% by weight, preferably less than 1 %, more preferably 0.1 % or less by weight of the stated ingredient. For example, the compositions of the invention that are substantially free of alcohol contain less than 3% by weight of alcohol, preferably less than 1% by weight of alcohol, more preferably 0.1% or less alcohol.

By the term "anti-microbial" we mean a compound or composition that kills and/or inhibits the growth of microbes (microorganisms). The term "microbiocidal" is used to refer to compounds or compositions that kill microbes. The compositions of the invention are anti-microbial and/or microbiocidal.

By the term "sanitising" or "sanitizer" we mean reducing the total number of microbes on a surface.

By the term "sterilising" we mean the elimination of microbiological organisms to achieve asepsis (a sterile microbial environment).

A microorganism or microbe is an organism that is microscopic (too small to be seen by the human eye). Examples of microorganisms include bacteria, fungi, yeasts, moulds, mycobacteria, algae spores, archaea and protists. Microorganisms are generally single-celled or unicellular organisms. However, as used herein, the term "microorganisms" also includes viruses.

The compositions of the invention may be anti-bacterial, anti-fungal, anti-algal, anti-sporal, anti-viral, anti-yeast and/or anti-mould.

The compositions of the invention are particularly suitable for use against bacteria, such as E.coli.

As used herein, the terms anti-bacterial, anti-fungal, anti-algal, anti-viral, anti-yeast and anti-mould agents are intended to refer to agents that inhibit the growth of the respective microorganisms but do not necessarily kill the microorganisms and agents that kill the respective microorganisms. Thus, for example, within the term anti-bacterial we include agents that inhibit the growth of bacteria but may not necessarily kill bacteria and bactericidal agents that do kill bacteria.

Examples of anti-bacterial agents include myobactericides and tuberculocides. Preferably, the compositions of the invention comprise at least one agent selected from anti-bacterial, anti-fungal, anti-algal, anti-sporal, anti-viral, anti-yeast and anti-mould agents and mixtures thereof. More preferably, the compositions of the invention comprise at least one anti-bacterial, anti-viral, anti-fungal and/or anti-mould agent.

The compositions of the invention can be effective against a wide range of organisms, including Gram negative and Gram positive bacteria, fungi, yeasts, viruses and some spore forming bacteria

An advantage of the invention is that it is possible to prevent a broad range of microorganisms from adhering and attaching to the surface, and, therefore, from forming a biofilm. Large numerous colonies are also substantially prevented from forming. Thus, the ability of the colony to grow is substantially reduced or even prevented. The invention is therefore general in its control of microorganisms.

In an aspect of the invention, the compositions may be used to reduce or control the formation of a biofilm.

As described previously, the compositions of the invention advantageously provide a residual anti-microbial effect whereby the anti-microbial action continues for a significant period after the initial application of the composition.

Typically, the compositions of the invention do not need to contain materials that are highly toxic to mammals. The anti-microbial agents used in the anti-microbial compositions are typically well known and widely understood and tested anti-microbial agents. The efficacy of the known anti-microbial agents is amplified in the formulations of the invention. Therefore, anti-microbial agents that have a low toxicity can be used in the anti-microbial compositions. In contrast, many "new" anti-microbial agents for known techniques of sanitization use "stronger", more toxic and/or little tested materials.

The anti-microbial compositions of the invention do not contain materials that produce highly persistent residues or rinsates or products that contain heavy metals and their salts. Thus, there is a greatly reduced risk of long term hazards.

The anti-microbial compositions of the invention do not interfere with the biochemical reproductive pathways of the micro-organisms they control. The risk of resistance build up and the development of resistant strains is, therefore, low.

The anti-microbial compositions of the invention can have a duel effect in that not only do they provide an anti-microbial effect in use but they can also have a preservative effect on the composition. This means that it is typically not necessary to include additional preservatives in the formulations of the invention.

The compositions of the invention do not typically give surfaces to which they are applied a greasy feel.

According to a further aspect of the invention, there is provided the use of composition of the invention to control, reduce or prevent the formation of colonies of micro-organisms on a surface at which it is provided.

The present invention provides a method for providing a residual anti-microbial benefit to a surface such as a hard surface or skin, which method comprises applying a composition as defined herein to that surface or skin. The composition may, for example, be applied to the surface or skin by spraying the composition on the surface or wiping the composition onto the surface or skin. In one method of the invention, it is not necessary for the method to include any steps in addition to simply applying the composition to the surface. Thus, a method that consists essentially of or consists of applying the composition to the surface or skin is provided.

In an aspect of the invention, the surface to which the composition of the invention is applied is a surface of the body, such as skin.

The anti-microbial compositions of the invention can typically degrade when submersed in water, to provide a rinsate/leachate of low toxicity and which has a short residence time in the environment.

According to a further aspect of the invention, there is provided the use of an anti-microbial composition of the invention to reduce or prevent the formation of colonies of microorganisms on a surface at which it is provided, such as skin.

The anti-microbial compositions of the invention are typically made by a process as described below.

A process for preparing an anti-microbial composition as herein described comprising:
(i) combining component (iii) and component (ii) with stirring; and
(ii) adding component (i) to components (iii) and (ii).

In some aspects, the pH of the solution obtained after step (ii) may require adjusting. Therefore, the present invention also describes a process in which (iii) the pH of the solution is adjusted after step (ii).

Preferences and options for a given aspect, feature or parameter of the invention should, unless the context indicates otherwise, be regarded as having been disclosed in combination with any and all preferences and options for all other aspects, features and parameters of the invention.

The present invention is illustrated by the following non-limiting Examples:

### Preparation of Test Examples and Comparative Examples

The compositions as described in Table 1 were prepared using the method of described below. The commercially available compounds used in the compositions are listed below.

Rewocid UTM 185 supplied by Evonik (REW): a 48% solution of [N(CH₃)₃(CH₂)₃NHC(O)(CH₂)₈CHCH₂]⁺ CH₃SO₄⁻, undecylen-amidopropyltrimonium methosulfate

*Acticide DDQ* 40 supplied by Thor (DDAC): contains 40% *di-n-decyldimethyl ammonium chloride* (DDAC). This is an example of component (ii) of the invention, being of Formula Type A.

*Acticide BAC 50M* supplied by Thor (BAC): contains 50% *Benzalkonium chloride* (BAC) is also an example of component (ii) of the invention, being of Formula Type B.

*Chlorhexidine di-gluconate (CHDG),* supplied by Evonik: a 20% solution is a further example of component (ii) of the invention.

*Benzethonium Chloride USP* supplied by Lonza (Benz): a Benzalkonium derivative and contains 100% Diisobutylphenoxyethoxyethyl-dimethylbenzyl ammonium chloride (BENZ) otherwise known as Benzethonium Chloride. It is a further example of component (ii) of the invention.

The polar solvent (component (iii)) was placed in a suitably sized vessel (typically of 1 litre capacity). A small amount of the polar solvent was retained to allow for final weight adjustment. The required amount of the biocide(s) (component (ii)), was then added to the vessel, with the vessel being stirred for 5 minutes after addition of each biocide before the next biocide is added. The required amount of the REW (component (i)) was then added and the vessel stirred for a further 5 minutes. The pH was checked and was adjusted using lactic acid or sodium hydroxide solution if required. The solution was then made up to 100% by weight by adding the retained polar solvent and stirred for a final 10 minutes.

The Comparative Examples were prepared following the same method but by leaving out one or more of the components (i) or (ii) of the invention.

In Table 1 the ppm of REW refers to the amount of the primary chemical component present in Rewocid UTM 185, namely undecylenamidopropyltrimonium methosulfate and the ppm of the components (ii) of the invention refers to the amount of the active chemical component, namely *DDAC-di-n-decyldimethyl ammonium chloride; CHDG-Chlorhexidine digluconate; BENZ-Benzethonium Chloride;* or *BAC-Benzalkonium Chloride.*

**Table 1**

| | **BENZ ppm** | **DDAC ppm** | **BAC ppm** | **CHDG ppm** | **REW ppm** | **(QUAT + CHDG) ppm** | **REW/(QUAT + CHDG)** |
|---|---|---|---|---|---|---|---|
| **Example 1** | 0 | 1800 | 0 | 600 | 600 | 2400 | 0.25 |
| **Example 2** | 0 | 1800 | 0 | 600 | 1200 | 2400 | 0.5 |
| **Example 3** | 0 | 1800 | 0 | 600 | 2400 | 2400 | 1 |
| **Example 4** | 0 | 1800 | 0 | 600 | 4800 | 2400 | 2 |
| **Example 5** | 0 | 0 | 1000 | 1000 | 4800 | 2000 | 2.4 |
| **Example 6** | 1000 | 0 | 0 | 1000 | 4800 | 2000 | 2.4 |
| **Example 7** | 0 | 1000 | 0 | 1000 | 4800 | 2000 | 2.4 |
| **Example 8** | 0 | 1800 | 0 | 600 | 7200 | 2400 | 3 |
| **Example 9** | 0 | 1800 | 0 | 600 | 9600 | 2400 | 4 |
| **Example 10** | 1800 | 0 | 0 | 600 | 19200 | 2400 | 8 |
| **Example 11** | 1800 | 0 | 0 | 600 | 24000 | 2400 | 10 |
| **Example 12** | 1800 | 0 | 0 | 600 | 36000 | 2400 | 15 |
| **Comparative Example 1** | 0 | 0 | 0 | 0 | 4800 | 0 | ∞ |
| **Comparative Example 2** | 0 | 2100 | 0 | 0 | 0 | 2100 | 0 |
| **Comparative Example 3** | 0 | 1800 | 0 | 600 | 0 | 2400 | 0 |
| **Comparative Example 4** | 0 | 0 | 1300 | 1300 | 0 | 2600 | 0 |
| **Comparative Example 5** | 0 | 0 | 1300 | 1000 | 0 | 2300 | 0 |
| **Comparative Example 6** | 0 | 0 | 0 | 2000 | 4800 | 2000 | 2.4 |
| **Comparative Example 7** | 1000 | 1000 | 0 | 0 | 1200 | 2000 | 0.6 |
| **Comparative Example 8** | 0 | 1250 | 0 | 350 | 0 | 1600 | 0 |
| **Comparative Example 9** | 0 | 1500 | 0 | 300 | 0 | 1800 | 0 |
| **Comparative Example 10** | 0 | 1750 | 0 | 250 | 0 | 2000 | 0 |
| **Comparative Example 11** | 0 | 1750 | 0 | 300 | 0 | 2050 | 0 |
| **Comparative Example 12** | 0 | 1750 | 0 | 350 | 0 | 2100 | 0 |

### Evaluation of Residual Anti-Microbial Efficacy of Examples and Comparative Examples

25 micro litres (0.025ml) of test sample was applied to a 2cm x 2cm section of Vitro-Skin (as supplied by IMS, Inc.) in duplicate in a Petri dish. The test sample was spread on the vitro-skin^{®} surface with a sterile inoculation loop ensuring that approximately a 0.5 cm perimeter was left untreated to the edge. The test sample was allowed to dry on the vitro-skin^{®}*₂ at 20°C in an oven with the Petri dish lid left off for one hour. The Petri dish was then removed from the oven and a strip of polypropylene wipe saturated with a sterile tryptone/saline solution placed inside. The lid was closed and then placed back in the oven for 4 hours at 20°C. After this time the Petri dish was removed from the oven and 10ul of a 1-1.5 x 10^8 cfu/ml. suspension of *E. coli* K12*₁ prepared in tryptone/saline solution was added to the surface of the vitro-skin^{®} containing the test sample and spread with a sterile inoculation loop ensuring the suspension stayed within the treated area of the vitroskin. The bacterial suspension was left in contact with the vitroskin for 5 minutes. After this time the vitro-skin^{®} was removed from the petri dish and placed in a test tube containing 10ml of neutralisation solution. It was shaken gently and left for a further 5 minutes after which the tube was vortex mixed for 1 minute to recover the bacteria from the surface of the vitroskin. A 1 ml sample of this suspension was then plated with tryptone soya agar and incubated for 48 hours at 37°C. After this time the log reduction in E. coli K12 was calculated by comparison with the water-treated control.

*₁ NB. *E. coli* K12 suspension prepared from a '2a' culture grown on tryptone soya agar for 24 hours at 37 °C, as described in BS EN microbiology standards e.g. EN1276:2009) *₂ vitro-skin^{®} is a testing substrate that mimics the surface properties of human skin. It is a synthetic non-biological product that has been formulated to have topography, pH, critical surface tension and ionic strength that are similar to human skin. It is a well-known and used substrate for those trained in the art to evaluate the benefits of topological applications of skin-care products to skin, which includes the evaluation of biocidal efficacy of actives applied to skin. Examples of published, peer-reviewed work with vitro-skin include US2009/0202463 which describes a method for evaluating antimicrobial compositions that provide enhanced immediate and residual anti-viral and antibacterial efficacy on the surface of skin; evaluation of the efficacy of organic acids in Hand Cleansers for the prevention of rhinovirus infections as described in the Journal of Antimicrobial Agents and Chemotherapy; 48(7); 2004 July; 2595-2598; US2012/0141396 describes disinfectant compositions that provide a prolonged antimicrobial property to a variety of surfaces, including skin, evaluated using vitro-skin.

The Examples and Comparative Examples were made to the compositions described in Table 1 following the method described above. The Examples and Comparative Examples Samples were then tested for anti-microbial efficacy using the method described above. The results of these tests are shown in Table 2 below.

**Table 2**

| | **Log R e-Coli vitro skin** |
|---|---|
| **Example 1** | > 4.24** |
| **Example 2** | > 5.14** |
| **Example 3** | > 4.24** |
| **Example 4** | > 4.19** |
| **Example 5** | > 5.23** |
| **Example 6** | > 5.23** |
| **Example 7** | > 5.23** |
| **Example 8** | > 4.24** |
| **Example 9** | > 4.24** |
| **Example 10** | > 4.24** |
| **Example 11** | > 4.19** |
| **Example 12** | > 4.19** |
| **Comparative Example 1** | 1.92 |
| **Comparative Example 2** | 2.48 |
| **Comparative Example 3** | 2.84 |
| **Comparative Example 4** | TNTC* |
| **Comparative Example 5** | TNTC* |
| **Comparative Example 6** | 2.86 |
| **Comparative Example 7** | 2.74 |
| **Comparative Example 8** | TNTC* |
| **Comparative Example 9** | TNTC* |
| **Comparative Example 10** | 3.39 |
| **Comparative Example 11** | 3.3 |
| **Comparative Example 12** | 3.59 |

| | |
|---|---|
| *TNTC = too numerous to count- Where the number of recoverable organisms post-testing on the recovery media exceeds the countable limits. This indicates that the organisms have largely remained unaffected in the test, and so the test sample has not performed well. ** Where a value is preceded by > it indicates Total Kill, this means that no bacterial colonies were present post-testing on the recovery media, meaning that the test sample has performed very well. | |

The results in Table 2 show that the compositions of the invention have superior residual anti-microbial performance compared to the Comparative Examples that are missing at least one feature of the compositions of the invention.

Comparative Example 1 shows that REW has almost no anti-microbial activity.

## Claims

1. An anti-microbial composition comprising:
(i) a compound of formula:
[N(CH₃)₃Q²]⁺Y⁻ (C²)
wherein Q² has structure -(CH₂)ₐNHC(O)(CH₂)_{b}CHCH₂, wherein a + b is an integer of from 8 to 18, provided that a and b are each independently an integer of from 1 to 9, wherein Y⁻ is a halide anion, alkylsulphate, sulphonate, saccharinate, or bicarbonate, or a mixture of compounds of formula (C²);
(ii) at least one anti-microbial quaternary ammonium compound and chlorhexidine or a chlorhexidine salt; and
(iii) a polar solvent.

2. A composition according to claim 1, wherein in the compound of formula (C²) a is from 1 to 5 and b is from 5 to 9.

3. A composition according to claim 1 or 2, wherein component (i) comprises [N(CH₃)₃(CH₂)₃NHC(O)(CH₂)₈CHCH₂]⁺ CH₃SO₄⁻, undecylenamidopropyltrimonium methosulfate.

4. A composition according to any one of the preceding claims, wherein component (ii) comprises an anti-microbial quaternary ammonium compound of formula (A) 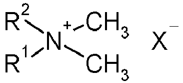 wherein R₁ and R₂ are each independently a straight chain, un-substituted and uninterrupted C₈₋₁₂ alkyl group and X⁻ is a halide anion such as chloride, bromide, fluoride, iodide or sulphonate, saccharinate, or bicarbonate and/or at least one benzalkonium compound of formula (B) 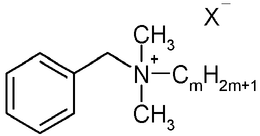 wherein m is from 8 to 18, optionally m is 8, 10 or 12, and X- is a halide anion such as chloride, bromide, fluoride, iodide or sulphonate, saccharinate, or bicarbonate; and/or benzethonium chloride.

5. A composition according to claim 4, wherein the benzalkonium compound of formula (B) is benzyldimethyl-n-tetradecyl-ammonium chloride, benzyldimethyl-n-dodecylammonium chloride, benzyl-C₁₂-C₁₆-alkyl-dimethyl-ammonium chloride, diisobutylphenoxyethoxyethyl-dimethylbenzyl ammonium chloride (otherwise known as Benzethonium Chloride) or benzyl-cocoalkyl-dimethyl-ammonium chloride and/or the compound of formula (A) is di-n-decyldimethyl ammonium chloride, octyl decyl dimethyl ammonium chloride or dioctyl dimethyl ammonium chloride,
optionally wherein the anti-microbial quaternary ammonium compound is selected from the group consisting of di-n-decyldimethyl ammonium chloride (DDAC), benzalkonium chloride (BAC), and benzethonium chloride (BENZ) and mixtures thereof.

6. A composition according to any one of the preceding claims comprising a quaternary ammonium compound of CAS number 7173-51-5 and/or 68424-85-1.

7. A composition according to claim 4, wherein the compound of formula (A) is di-n-decyldimethyl ammonium chloride, octyl decyl dimethyl ammonium chloride or dioctyl dimethyl ammonium chloride or a mixture thereof, optionally wherein the compound of formula (A) is di-n-decyldimethyl ammonium chloride, and wherein the composition optionally additionally comprises a compound of formula (B) as defined in any one of claims 4 or 5.

8. A composition according to any one of the preceding claims, wherein the chlorhexidine is chlorhexidine di-gluconate.

9. A composition according to any one of the preceding claims, wherein
(a) the amount of component (i) is from about 0.01 to about 5% by weight of the composition or from 0.05 to about 2% by weight of the composition, optionally wherein the amount of component (i) is from about 0.08 to about 0.5% by weight of the composition; and/or
(b) the total amount of component (ii), and if present an additional antimicrobial component (b) is from about 0.01 to about 5% by weight of the composition or from about 0.02 to about 2% by weight of the composition, optionally wherein the total amount of component (ii) and component (b) if present is from about 0.05 to about 0.6% by weight of the composition; and/or
(c) the ratio of component (i) to component (ii) is from 15:1 to 1:10 or from 5:1 to 1:5; and/or
(d) the ratio of the at least one anti-microbial quaternary ammonium compound to chlorhexidine or a chlorhexidine salt is from 1:1 to 3:1.

10. A composition according to any one of the preceding claims, wherein
(a) the pH of the composition is from about 4 to about 7; and/or
(b) the polar solvent is selected from water, ethanol, n-propanol, isopropanol, ethylene glycol ethers, propylene glycol ethers, butyl diglycol (BDG) and dipropylene glycol methyl ether (Trade name Dowanol DPM) and mixtures thereof; and/or
(c) the composition is substantially free of alcohol.

11. A composition according to any one of the preceding claims, wherein the ratio of the at least one anti-microbial quaternary ammonium compound to chlorhexidine or a chlorhexidine salt is from 1:1 to 3:1 and the ratio of component (i) to component (ii) is from 15:1 to 1:10 or from 5:1 to 1:5;
optionally wherein the amount of component (i) is from about 0.01 to about 5% by weight of the composition and the total amount of component (ii), and component (b) if present is from about 0.05 to about 0.6% by weight of the composition.

12. The non-therapeutic use of a composition according to any one of the preceding claims to substantially reduce or control the formation of microbial colonies on or at a surface, wherein the surface is selected from hard surfaces optionally selected from bathroom surfaces, kitchen surfaces, living area surfaces, hard floors, carpets, furniture, glass/mirrors, toilet surfaces, outdoor surfaces such as wood, stone, concrete or plastics, BBQ surfaces, walls, fences; plant surfaces and food surfaces, optionally wherein the use comprises reducing or controlling the formation of biofilms.

13. A non-therapeutic method of substantially reducing or controlling the formation of microbial colonies on or at a surface, wherein the surface is selected from hard surfaces optionally selected from bathroom surfaces, kitchen surfaces, living area surfaces, hard floors, carpets, furniture, glass/mirrors, toilet surfaces, outdoor surfaces such as wood, stone, concrete or plastics, BBQ surfaces, walls, fences; plant surfaces and food surfaces, which method comprises applying a composition according to any one of claims 1 to 11 to that surface, optionally wherein the method is to reduce or control the formation of biofilms.

14. A non-therapeutic method of disrupting, preventing or reducing the adhesion and/or attachment of micro-organisms to a surface, wherein the surface is selected from hard surfaces optionally selected from bathroom surfaces, kitchen surfaces, living area surfaces, hard floors, carpets, furniture, glass/mirrors, toilet surfaces, outdoor surfaces such as wood, stone, concrete or plastics, BBQ surfaces, walls, fences; plant surfaces and food surfaces; which method comprises applying a composition according to any one of claims 1 to 11 to that surface.

15. A hand sanitizer, wound care product or anti-microbial wipe comprising an anti-microbial composition as defined in any one of claims 1 to 11.

16. A composition according to any one of claims 1 to 11 for use to reduce or control the formation of microbial colonies on the skin or a wound; optionally wherein the use comprises the reducing or controlling the formation of biofilms.

17. A composition according to any one of claims 1 to 11 for use in a method of preventing or reducing the adhesion and/or attachment of micro-organisms to the skin.

## Patentansprüche

1. Antimikrobielle Zusammensetzung, umfassend:
(i) eine Verbindung der Formel:
[N(CH₃)₃Q²]⁺Y⁻ (C²)
wobei Q² die Struktur -(CH₂)ₐNHC(O)(CH₂)_{b}CHCH₂ aufweist, wobei a + b eine ganze Zahl von 8 bis 18 ist, mit der Maßgabe, dass a und b jeweils unabhängig voneinander eine ganze Zahl von 1 bis 9 sind, wobei Y⁻ ein Halogenidanion, Alkylsulfat, Sulfonat, Saccharinat oder Bicarbonat ist, oder eine Mischung von Verbindungen der Formel (C²);
(ii) mindestens eine antimikrobielle quaternäre Ammoniumverbindung und Chlorhexidin oder ein Chlorhexidinsalz; und
(iii) ein polares Lösungsmittel.

2. Zusammensetzung nach Anspruch 1, wobei in der Verbindung der Formel (C²) a von 1 bis 5 und b von 5 bis 9 ist.

3. Zusammensetzung nach Anspruch 1 oder 2, wobei Komponente (i) [N(CH₃)₃(CH₂)₃NHC(O)(CH₂)₈CHCH₂]⁺ CH₃SO₄⁻, Undecylenamidopropyltrimoniummethosulfat, umfasst.

4. Zusammensetzung nach einem der vorangehenden Ansprüche, wobei Komponente (ii) eine antimikrobielle quaternäre Ammoniumverbindung der Formel (A) 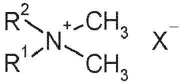 umfasst,
wobei R₁ und R₂ jeweils unabhängig voneinander eine geradkettige, unsubstituierte und ununterbrochene C₈₋₁₂-Alkylgruppe sind und X⁻ ein Halogenidanion, wie z.B. Chlorid, Bromid, Fluorid, Iodid oder Sulfonat, Saccharinat oder Bicarbonat ist, und/oder mindestens eine Benzalkoniumverbindung der Formel (B) 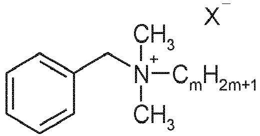 ist,
wobei m 8 bis 18 ist, optional m 8, 10 oder 12 ist und X⁻ ein Halogenidanion wie Chlorid, Bromid, Fluorid, Iodid oder Sulfonat, Saccharinat oder Bicarbonat ist; und/oder Benzethoniumchlorid.

5. Zusammensetzung nach Anspruch 4, wobei die Benzalkoniumverbindung der Formel (B) Benzyldimethyl-n-tetradecyl-ammoniumchlorid, Benzyldimethyl-n-dodecyl-ammoniumchlorid, Benzyl-C₁₂-C₁₆-alkyl-dimethyl-ammonium-chlorid, Diisobutylphenoxyethoxyethyl-dimethylbenzyl-ammoniumchlorid (auch bekannt als Benzethoniumchlorid) oder Benzyl-cocoalkyl-dimethylammoniumchlorid ist und/oder die Verbindung der Formel (A) Di-n-decyldimethylammoniumchlorid, Octyldecyldimethyl-ammoniumchlorid oder Dioctyldimethylammoniumchlorid ist,
wobei optional die antimikrobielle quaternäre Ammoniumverbindung aus der Gruppe ausgewählt ist, die aus Di-n-decyldimethylammoniumchlorid (DDAC), Benzalkoniumchlorid (BAC) und Benzethoniumchlorid (BENZ) und Mischungen dieser besteht.

6. Zusammensetzung nach einem der vorangehenden Ansprüche, umfassend eine quaternäre Ammoniumverbindung mit der CAS-Nummer 7173-51-5 und/oder 68424-85-1.

7. Zusammensetzung nach Anspruch 4, wobei es sich bei der Verbindung der Formel (A) um Di-n-decyldimethylammoniumchlorid, Octyldecyldimethyl-ammoniumchlorid oder Dioctyldimethylammoniumchlorid oder eine Mischung dieser handelt, wobei es sich bei der Verbindung der Formel (A) optional um Di-n-decyldimethylammoniumchlorid handelt, und wobei die Zusammensetzung optional zusätzlich eine Verbindung der Formel (B), wie in einem der Ansprüche 4 oder 5 definiert, umfasst.

8. Zusammensetzung nach einem der vorangehenden Ansprüche, wobei das Chlorhexidin Chlorhexidin-di-gluconat ist.

9. Zusammensetzung nach einem der vorangehenden Ansprüche, wobei
(a) die Menge der Komponente (i) von etwa 0,01 bis etwa 5 Gew.-% der Zusammensetzung oder von 0,05 bis etwa 2 Gew.-% der Zusammensetzung beträgt, wobei die Menge der Komponente (i) optional von etwa 0,08 bis etwa 0,5 Gew.- % der Zusammensetzung beträgt; und/oder
(b) die Gesamtmenge der Komponente (ii) und, falls vorhanden, einer zusätzlichen antimikrobiellen Komponente (b) von etwa 0,01 bis etwa 5 Gew.-% der Zusammensetzung oder von etwa 0,02 bis etwa 2 Gew.-% der Zusammensetzung beträgt, wobei optional die Gesamtmenge der Komponente (ii) und der Komponente (b), falls vorhanden, von etwa 0,05 bis etwa 0,6 Gew.-% der Zusammensetzung beträgt; und/oder
(c) das Verhältnis von Komponente (i) zu Komponente (ii) von 15:1 bis 1:10 oder von 5:1 bis 1:5 beträgt; und/oder
(d) das Verhältnis der mindestens einen antimikrobiellen quaternären Ammoniumverbindung zu Chlorhexidin oder einem Chlorhexidinsalz von 1:1 bis 3:1 beträgt.

10. Zusammensetzung nach einem der vorangehenden Ansprüche, wobei
(a) der pH-Wert der Zusammensetzung von etwa 4 bis etwa 7 beträgt; und/oder
(b) das polare Lösungsmittel ausgewählt ist aus Wasser, Ethanol, n-Propanol, Isopropanol, Ethylenglykolethern, Propylenglykolethern, Butyldiglykol (BDG) und Dipropylenglykolmethylether (Handelsname Dowanol DPM) und Mischungen davon; und/oder
(c) die Zusammensetzung im Wesentlichen frei ist von Alkohol .

11. Zusammensetzung nach einem der vorangehenden Ansprüche, wobei das Verhältnis der mindestens einen antimikrobiellen quaternären Ammoniumverbindung zu Chlorhexidin oder einem Chlorhexidinsalz 1:1 bis 3:1 beträgt und das Verhältnis von Komponente (i) zu Komponente (ii) von 15:1 bis 1:10 oder von 5:1 bis 1:5 beträgt; wobei optional die Menge der Komponente (i) von etwa 0,01 bis etwa 5 Gew.-% der Zusammensetzung beträgt und die Gesamtmenge der Komponente (ii) und der Komponente (b), falls vorhanden, von etwa 0,05 bis etwa 0,6 Gew.-% der Zusammensetzung beträgt.

12. Nicht-therapeutische Verwendung einer Zusammensetzung nach einem der vorangehenden Ansprüche, um die Bildung von mikrobiellen Kolonien auf oder an einer Oberfläche im Wesentlichen zu reduzieren oder zu kontrollieren, wobei die Oberfläche ausgewählt ist aus harten Oberflächen, optional ausgewählt aus Badezimmeroberflächen, Küchenoberflächen, Wohnbereichs-oberflächen, harten Böden, Teppichen, Möbeln, Glas/Spiegeln, Toilettenoberflächen, Außenoberflächen wie Holz, Stein, Beton oder Kunststoffen, Grilloberflächen, Wänden, Zäunen; Pflanzenoberflächen und Lebensmitteloberflächen, wobei die Verwendung optional das Reduzieren oder Kontrollieren der Bildung von Biofilmen umfasst.

13. Nicht-therapeutisches Verfahren zum wesentlichen Verringern oder Kontrollieren der Bildung von mikrobiellen Kolonien auf oder an einer Oberfläche, wobei die Oberfläche ausgewählt ist aus harten Oberflächen, optional ausgewählt aus Badezimmeroberflächen, Küchenoberflächen, Wohnbereichsoberflächen, harten Böden, Teppichen, Möbeln, Glas/Spiegeln, Toiletten-oberflächen, Außenoberflächen wie Holz, Stein, Beton oder Kunststoffen, Grilloberflächen, Wänden, Zäunen; Pflanzenoberflächen und Lebensmitteloberflächen, wobei das Verfahren das Auftragen einer Zusammensetzung gemäß einem der Ansprüche 1 bis 11 auf diese Oberfläche umfasst, wobei das Verfahren optional das Verringern oder Kontrollieren der Bildung von Biofilmen ist.

14. Nicht-therapeutisches Verfahren zum Stören, Verhindern oder Verringern der Adhäsion und/oder Anhaftung von Mikroorganismen an einer Oberfläche, wobei die Oberfläche ausgewählt ist aus harten Oberflächen, die optional ausgewählt sind aus Badezimmeroberflächen, Küchenoberflächen, Wohnbereichsoberflächen, harten Böden, Teppichen, Möbeln, Glas/Spiegeln, Toiletten-oberflächen, Außenoberflächen wie Holz, Stein, Beton oder Kunststoffen, Grilloberflächen, Wänden, Zäunen; Pflanzenoberflächen und Lebensmitteloberflächen, wobei das Verfahren das Auftragen einer Zusammensetzung gemäß einem der Ansprüche 1 bis 11 auf diese Oberfläche umfasst.

15. Handdesinfektionsmittel, Wundpflegeprodukt oder antimikrobielles Wischtuch, umfassend eine antimikrobielle Zusammensetzung gemäß einem der Ansprüche 1 bis 11.

16. Zusammensetzung nach einem der Ansprüche 1 bis 11 zur Verwendung zur Verringerung oder Kontrolle der Bildung von mikrobiellen Kolonien auf der Haut oder einer Wunde; wobei die Verwendung optional die Verringerung oder Kontrolle der Bildung von Biofilmen umfasst.

17. Zusammensetzung nach einem der Ansprüche 1 bis 11 zur Verwendung in einem Verfahren zur Verhinderung oder Verringerung der Adhäsion und/oder Anhaftung von Mikroorganismen an der Haut.

## Revendications

1. Composition anti-microbienne comprenant :
(i) un composé de formule :
[N(CH₃)₃Q²]⁺Y⁻ (C²)
dans laquelle Q² a la structure - (CH₂)ₐNHC(O)(CH₂)_{b}CHCH₂, dans laquelle a + b est un entier de 8 à 18, à condition que a et b soient chacun indépendamment un entier de 1 à 9, dans laquelle Y⁻ est un anion halogénure, alkylsulfate, sulfonate, saccharinate ou bicarbonate, ou un mélange de composés de formule (C²) ;
(ii) au moins un composé d'ammonium quaternaire antimicrobien et de la chlorhexidine ou un sel de chlorhexidine ; et
(iii) un solvant polaire.

2. Composition selon la revendication 1, dans laquelle dans le composé de formule (C²) a vaut de 1 à 5 et b vaut de 5 à 9.

3. Composition selon la revendication 1 ou 2, dans laquelle le composant (i) comprend [N(CH₃)₃(CH₂)₃NHC(O)(CH₂)₈CHCH₂]⁺ CH₃SO₄⁻, le méthosulfate d'undécylénamidopropyltrimonium.

4. Composition selon l'une quelconque des revendications précédentes, dans laquelle le composant (ii) comprend un composé d'ammonium quaternaire antimicrobien de formule (A). 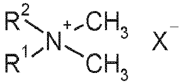 dans laquelle R₁ et R₂ sont chacun indépendamment un groupe alkyle en C₈₋₁₂ à chaîne droite, non substitué et ininterrompu et X⁻ est un anion halogénure tel que chlorure, bromure, fluorure, iodure ou sulfonate, saccharinate ou bicarbonate et/ou au moins un composé de benzalkonium de formule (B) 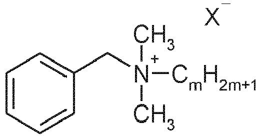 dans laquelle m vaut de 8 à 18, éventuellement m vaut 8, 10 ou 12, et X- est un anion halogénure tel que chlorure, bromure, fluorure, iodure ou sulfonate, saccharinate ou bicarbonate ; et/ou chlorure de benzéthonium.

5. Composition selon la revendication 4, dans laquelle le composé de benzalkonium de formule (B) est le chlorure de benzyldiméthyl-n-tétradécyl-ammonium, le chlorure de benzyldiméthyl-n-dodécyl-ammonium, le chlorure de benzyl-(alkyl en C₁₂-C₁₆)-diméthyl-ammonium, le chlorure de diisobutylphénoxyéthoxyéthyl-diméthylbenzylammonium (connu par ailleurs sous le nom de chlorure de benzéthonium) ou le chlorure de benzyl-cocoalkyl-diméthyl-ammonium et/ou le composé de formule (A) est le chlorure de di-n-décyldiméthylammonium, le chlorure d'octyldécyldiméthylammonium ou le chlorure de dioctyldiméthylammonium,
éventuellement dans laquelle le composé d'ammonium quaternaire antimicrobien est choisi dans le groupe constitué du chlorure de di-n-décyldiméthylammonium (DDAC), du chlorure de benzalkonium (BAC) et du chlorure de benzéthonium (BENZ) et des mélanges de ceux-ci.

6. Composition selon l'une quelconque des revendications précédentes comprenant un composé d'ammonium quaternaire de numéro CAS 7173-51-5 et/ou 68424-85-1.

7. Composition selon la revendication 4, dans laquelle le composé de formule (A) est le chlorure de di-n-décyldiméthylammonium, le chlorure d'octyldécyldiméthylammonium ou le chlorure de dioctyldiméthylammonium ou un mélange de ceux-ci, éventuellement dans laquelle le composé de formule (A) est le chlorure de di-n-décyldiméthylammonium, et dans laquelle la composition comprend éventuellement en outre un composé de formule (B) tel que défini dans l'une quelconque des revendications 4 ou 5.

8. Composition selon l'une quelconque des revendications précédentes, dans laquelle la chlorhexidine est le digluconate de chlorhexidine.

9. Composition selon l'une quelconque des revendications précédentes, dans laquelle
(a) la quantité de composant (i) est d'environ 0,01 à environ 5 % en poids de la composition ou de 0,05 à environ 2 % en poids de la composition, éventuellement dans laquelle la quantité de composant (i) est d'environ 0,08 à environ 0,5 % en poids de la composition ; et/ou
(b) la quantité totale de composant (ii), et s'il est présent de composant antimicrobien supplémentaire (b) est d'environ 0,01 à environ 5 % en poids de la composition ou d'environ 0,02 à environ 2 % en poids de la composition, éventuellement dans laquelle la quantité totale de composant (ii) et de composant (b) s'il est présent est d'environ 0,05 à environ 0,6 % en poids de la composition ; et/ou
(c) le rapport du composant (i) au composant (ii) est de 15:1 à 1:10 ou de 5:1 à 1:5 ; et/ou
(d) le rapport de l'au moins un composé d'ammonium quaternaire antimicrobien à la chlorhexidine ou à un sel de chlorhexidine est de 1:1 à 3:1.

10. Composition selon l'une quelconque des revendications précédentes, dans laquelle
(a) le pH de la composition est d'environ 4 à environ 7 ; et/ou
(b) le solvant polaire est choisi parmi l'eau, l'éthanol, le n-propanol, l'isopropanol, les éthers d'éthylène glycol, les éthers de propylène glycol, le méthyléther de butyl diglycol (BDG) et de dipropylène glycol (nom commercial Dowanol DPM) et des mélanges de ceux-ci ; et/ou
(c) la composition est sensiblement exempte d'alcool.

11. Composition selon l'une quelconque des revendications précédentes, dans laquelle le rapport de l'au moins un composé d'ammonium quaternaire antimicrobien à la chlorhexidine ou à un sel de chlorhexidine est de 1:1 à 3:1 et le rapport du composant (i) au composant (ii) est de 15:1 à 1:10 ou de 5:1 à 1:5 ;
éventuellement dans laquelle la quantité de composant (i) est d'environ 0,01 à environ 5 % en poids de la composition et la quantité totale de composant (ii), et de composant (b) s'il est présent est d'environ 0,05 à environ 0,6 % en poids de la composition.

12. Utilisation non thérapeutique d'une composition selon l'une quelconque des revendications précédentes pour réduire sensiblement ou contrôler la formation de colonies microbiennes sur ou au niveau d'une surface, dans laquelle la surface est choisie parmi des surfaces dures éventuellement choisies parmi des surfaces de salle de bain, des surfaces de cuisine, des surfaces de salon, des sols durs, des tapis, des meubles, du verre/des miroirs, des surfaces de toilettes, des surfaces extérieures telles que le bois, la pierre, le béton ou le plastique, des surfaces de barbecue, des murs, des clôtures ; des surfaces végétales et des surfaces alimentaires, éventuellement dans laquelle l'utilisation comprend la réduction ou le contrôle de la formation de biofilms.

13. Procédé non thérapeutique de réduction substantielle ou de contrôle de la formation de colonies microbiennes sur ou au niveau d'une surface, dans lequel la surface est choisie parmi des surfaces dures éventuellement choisies parmi des surfaces de salle de bain, des surfaces de cuisine, des surfaces de salon, des sols durs, des tapis, des meubles, du verre/des miroirs, des surfaces de toilettes, des surfaces extérieures telles que le bois, la pierre, le béton ou le plastique, des surfaces de barbecue, des murs, des clôtures ; des surfaces végétales et des surfaces alimentaires, lequel procédé comprend l'application d'une composition selon l'une quelconque des revendications 1 à 11 sur cette surface, éventuellement dans lequel le procédé consiste à réduire ou contrôler la formation de biofilms.

14. Procédé non thérapeutique pour perturber, empêcher ou réduire l'adhésion et/ou la fixation de micro-organismes à une surface, dans lequel la surface est choisie parmi des surfaces dures éventuellement choisies parmi des surfaces de salle de bain, des surfaces de cuisine, des surfaces de salon, des sols durs, des tapis, des meubles, du verre/des miroirs, des surfaces de toilettes, des surfaces extérieures telles que le bois, la pierre, le béton ou le plastique, des surfaces de barbecue, des murs, des clôtures ; des surfaces végétales et des surfaces alimentaires ; lequel procédé comprend l'application d'une composition selon l'une quelconque des revendications 1 à 11 sur cette surface.

15. Désinfectant pour les mains, produit de soin des plaies ou lingette antimicrobienne comprenant une composition antimicrobienne telle que définie dans l'une quelconque des revendications 1 à 11.

16. Composition selon l'une quelconque des revendications 1 à 11 destinée à être utilisée pour réduire ou contrôler la formation de colonies microbiennes sur la peau ou une plaie ; éventuellement dans laquelle l'utilisation comprend la réduction ou le contrôle de la formation de biofilms.

17. Composition selon l'une quelconque des revendications 1 à 11 destinée à être utilisée dans un procédé de prévention ou de réduction de l'adhésion et/ou de la fixation de micro-organismes sur la peau.
